# EUROPEAN PATENT SPECIFICATION

(11) **EP 4 041 232 B1**
(45) Date of publication and mention of the grant of the patent: **19.07.2023**
(21) Application number: 20821364.5
(22) Date of filing: 06.10.2020
(51) Int. Cl.: A61K 31/4545, A61K 45/06, A61P 25/00, A61J 3/07

(54) **APPARATUS FOR OPENING AND CLOSING OF TWO-PART MATERIAL CONTAINERS, PREFERABLY CAPSULES**
VORRICHTUNG ZUM ÖFFNEN UND SCHLIESSEN VON ZWEITEILIGEN MATERIALBEHÄLTERN, VORZUGSWEISE KAPSELN
APPAREIL POUR OUVRIR ET FERMER DES RÉCIPIENTS EN MATÉRIAU EN DEUX PARTIES, DE PRÉFÉRENCE DES CAPSULES

(30) Priority: 07.10.2019 HU 1900190 U
(43) Date of publication of application: 17.08.2022
(73) Proprietor: Innogamma Kft., 2862 Aka (HU)
(72) Inventor: HADAS, Tamás, 1086 Budapest (HU)
(74) Representative: Danubia Patent & Law Office LLC
(86) International application number: PCT/HU2020/050045
(87) International publication number: WO 2021/069941

(56) References cited:
- DE-U1-202019 001 756
- US-A- 3 501 894
- US-A- 3 587 671
- US-A- 4 163 354

## Description

The invention relates to an apparatus for opening and closing two-part material containers, preferably capsules, comprising a sleeve-like housing comprising an open first end and a closed second end; a seat seizing the lower part of a capsule arranged slideably along the longitudinal axis of the housing, and a seat gripping the upper part of a capsule that can be introduced at the open end of the housing during the operation of the apparatus and, furthermore, an actuator moving the lower part and the upper part of the capsule coaxially with the housing and along its longitudinal axis, in releasable connection with the seat gripping the upper part.

Capsules made of gelatine or metal are widely used for the storage in dosed quantitiesof various material preparations, thus radiant isotopes used for pharmaceuticals and industrial radiation sources. These can be filled with material preparations in several ways.

Capsules manufactured in large quantities, containing pharmaceuticals products, are filled by automatic or semi-automatic devices. One example is disclosed in the document GB2571226. Industrial automatic capsule filling machines, however, are not suitable for closing capsules of a small quantity, because their setting and operation is not economical below a given quantity. Other examples can be found in US3501894, DE202019001756, US4163354, or US3587671.

In the case of capsule products made in smaller quantities, the capsules are usually filled and then closed manually. The description of the utility model TW M578585, for example, discloses a capsule filling device comprising a stand, a filling device that can be inserted into the upper part of the stand, and a lower part that can be inserted under the stand to receive the material charge. This solution, however, does not allow to close the already filled capsules.

The solution disclosed in utility model TW M349774, on the other hand, isalready suitable for closing capsules. It essentially contains blocks with slots for simultaneous receiving capsule lower parts, so called bodies and upper parts, so called caps, with openings under the slots and holding sticks extending into the slots through the openings located under the block. The upper part of the capsule is inserted manually into the slots and, after the insertion of the upper parts of the capsules, the capsules are filled with the material preparation concerned by using another device. Then, the lower capsule parts are dropped into the slots manually. Finally, a sheet is laid upon the upper part of the block, and pressure is exerted on it. Under the impact of the pressure, the block moves downwards and thus the two capsule halves fitted between the sheet and the holding stick are compressed together and consequently closed.

A drawback of this solution is that the entire process is realised by manual force, which makes the process very slow on the one hand. On the other hand, with material preparations containing ingredients that are adverse or harmful to health, e.g. that emit radioactive radiation, it is difficult and costly to design a workstation appropriate also from the point of view of health safety.

A further drawback is that the use of a substantial amount of live labour also makes the production of such closed capsules expensive. Furthermore, disposing of the contaminated equipment after deterioration can also be a problem.

Yet another drawback is that in case of manual capsule closing with the help of the device concerned - where the lower and upper parts of the capsule are arranged in the same place, on top of each other, occasionally in a positioned way - the respective edges of the lower and upper parts of the capsule may be damaged or may bend, resulting in scrap, which further deteriorates the efficiency of capsule closing process.

Besides the above, it is also considered a drawback that the empty capsules to be used - the relevant capsule halves being usually provided placed upon one another, but not closed - are to be opened in a separated step before the operations outlined above, and may require substantial energy and time.

The object of the invention is to create an apparatus that is suitable for being connected to a programmable robot and is thus capable of opening capsules, i.e. quickly separating the capsule halves and closing capsule halves containing any filling, in a quick, accurate and damage-free way, even without special human intervention.

To that end, my aim is to have an apparatus of a simple design, capable of the damage-free separation and then accurate closing of the upper and lower part of a capsule, so that no human contribution be needed for either the opening or the filling or closing operations.

The recognition leading to the novel design was that by complementing the supporting stud holding the capsule lower part from below by a special, purpose-oriented orienting component and a gripping unit cooperating with it, which surrounds the lower part well, but has a different design and function than the known ones and, moreover, a novel-type structural element, independent of the component holding the lower part and independently moveable along at least two axes, is used also for gripping the capsule upper part, that is capable of cooperating precisely with the orienting component complementing the supporting stud through its special design, we can automatically remove the upper part from the lower part of the capsule insertable into the orienting component, realising a complete capsule opening process; then, after loading with the capsule with the material preparation, the capsule upper part acting as cover that can be inserted appropriately into the independently moveable structural element mentioned above can be transported to above the lower part so that orienting and guiding together the parts is very precise throughout the closing operation and thus the entire capsule closing process can be accomplished quickly and precisely without human contribution.

The task outlined above has been solved by a two-part material container preferably for opening and closing a capsule, comprising a sleeve-like housing comprising an open first end and a closed second end; a seat seizing the lower part of a capsule arranged slideably along the longitudinal axis of the housing, and a seat gripping the upper part of a capsule that can be introduced at the open end of the housing during the operation of the apparatus and, furthermore, an actuator moving the lower part and the upper part of the capsule coaxially with the housing and along its longitudinal axis, in releasable connection with the seat gripping the upper part. In a novel way a flexible element counter-holding the seat against the open end of the sleeve-like housing is inserted between the closed end of the sleeve-like housing and the seat gripping the lower part of the capsule; the seat gripping the lower part of the capsule comprises a centre chamber open towards the closed end of the sleeve-like housing; an actuator supporting the capsule lower part is inserted into the capsule-seizing seat at the closed end of the sleeve-like housing is embedded rotatably and so that it extends into the centre chamber of the seat gripping the lower part of the capsule; in the central cavity of the seat gripping the lower part of the capsule, a flexible clamping means fixing the lower part of the inserted capsule temporarily is arranged; a sleeve is mounted unrotatably onto the rotatable actuating member, so that it can be pushed along the length of the actuating member; the front surface of one end of the sleeve is connected to the flexible clamping means; the front surface of the other end of the sleeve contains at least one diametrically formed wavy projection, and at least one forcing means is formed, pushing the sleeve on the actuating member's supporting stud and consequently pressing the flexible clamping meansonto the capsule lower part inserted into the centre opening of the seat gripping the capsule lower part through the rotation of the actuating member and the sleeve relative to each other.

A further preferred characteristic of the apparatus according to the invention could be that the flexible component supporting the seat in the direction of the open end of the sleeve-like housing is a cylindrical spring.

In a further preferred embodiment of the apparatus, the support stud supporting the capsule lower part comprises a centring seat radially centralising the lower part of the capsule.

In a further preferred embodiment of the apparatus, the flexible clamping means is an O ring.

In another preferred embodiment of the apparatus, the forcing means formed on the actuating member is a bolt in connection with the front surface of the sleeve containing a wavy projection, arranged diametrically on the actuating member and, in a further preferred embodiment, the clamping means designed on the actuating member consists of two bearings in connection with the front surface of the sleeve containing a wavy projection, arranged diametrically on the actuating member.

In a further preferred embodiment of the apparatus, the open end of the sleeve-like housing is designed with bevelling introducing the seat gripping the upper part of the capsule.

Another preferred characteristic of the apparatus according to the invention could be that it comprises a vacuum unit holding the capsule upper part inserted in the seat gripping the capsule upper part that is connected to the seat gripping the capsule upper part.

According to a further preferred characteristic of the apparatus according to the invention, the actuating member comprises an ejection unit moving the capsule upper part inserted in the capsule upper part holding seat out of the seat.

In a further preferred embodiment of the apparatus according to the invention, the ejection unit comprises an ejection rod connected to a syringe plunger constituting the vacuum unit that is guided concentrically with the duct providing the connection.

The apparatus according to the invention has several advantageous characteristics. The most important of these is that, thanks to the novel-type seat and the guiding house cooperating with the actuating member, and the also unusual special upper seat that can be positioned with the housing, but can be moved completely independently of the lower seat, even in 3D, the lower part and the upper part of the capsule can be closed quickly and precisely, even with a 3D programmed robot, without the touch of the human hand, so that dosing the filling into the lower part and picking up the upper part from a different place and delivering it to above the lower part can also be done by a robot, something that had not been feasible before.

A further advantage coming from the above is that automation and, consequently, cheaper, yet highly precise closing orientation is feasible even for material containers, e.g. capsules, made in a small quantity. As a result, no live labour required and, furthermore, precise guiding excludes the possibility of any damage to the capsule edges, thus basically no scrap is generated.

The elimination of the need for live labour is a particularly substantial advantage in the manufacture of radio-pharmaceuticals capsules and the preparation of radioactive sources. Using the apparatus according to the invention makes it possible to use the robot mentioned already, so the personnel is not exposed to radiation, since in addition to dosage, the closing of the individual capsules and containers is also done by the robot.

Another major advantage is that the interior design of the seat receiving the lower part and its "connection" with the seat receiving the upper part during closing and their joint sinking creates the advantage that the external surface of the capsule parts is well surrounded throughout the process of the closing of the capsule parts, and the length of the compression path is also well-regulated. Thus the upper part of the capsule slides precisely, at appropriate depth, onto the lower part of the capsule, ensuring the good closing of the capsule in every case. This is of high relevance again if radioactive materials are treated.

Given its small dimensions and mass, the apparatus according to the invention can be advantageously used also with a small robot, which makes it possible to use it in protected or closed spaces, e.g. in a hot booth or a laminar booth, promoting the elimination of live labour at such places and protecting the personnel from e.g. possible radiation.

In the following, the apparatus according to the invention is disclosed in more detail based on an exemplary embodiment, referring to the drawings. In the drawings,
- Figure 1: shows a longitudinal section of a possible embodiment of the apparatus according to the invention connected to an actuating member, with an inserted capsule,
- Figure 2: shows the longitudinal section of the apparatus according to Figure 1, rotated by 90°, comprising an activated actuating member,
- Figure 3: shows a magnified perspective view of the actuating member used in the apparatus according to Figure 1,
- Figure 4: shows a magnified perspective view of the sleeve used in the apparatus according to Figure 1,
- Figure 5: shows the longitudinal section of the sleeve used in the apparatus according to Figure 1, and
- Figure 6: shows the apparatus according to Figure 1 in the position following final closing of the inserted capsule, in longitudinal section.

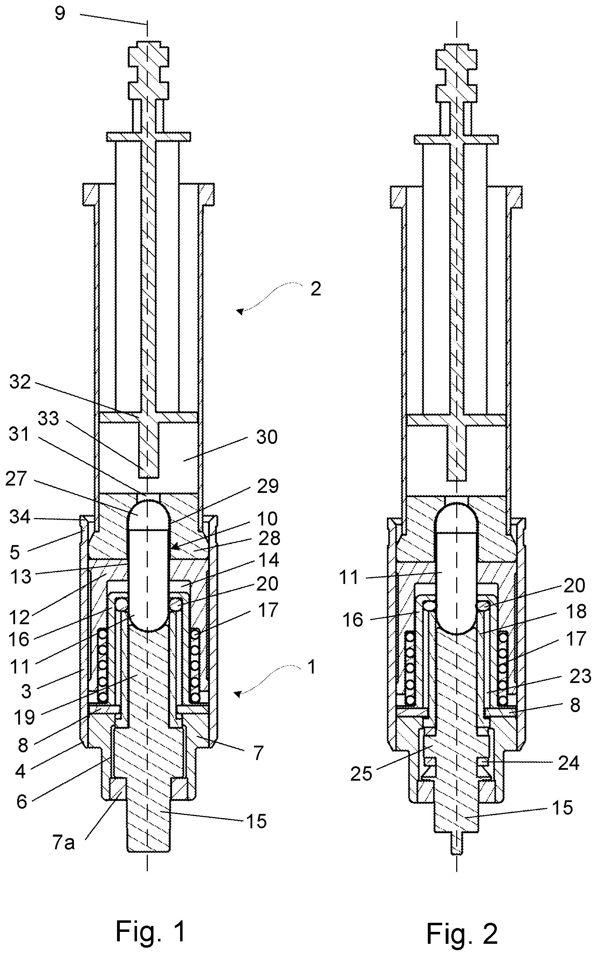

An apparatus 1 shown in the figures, considered only a preferred embodiment, is shown in the present case together with a separate complementing means in Figure 1. Apparatus 1 according to the invention is visible in the lower part of the figure, whereas in the upper part of the figure one can observe an actuator 2 being connected there to the apparatus, the structure and function of which will be explained in detail later.

Apparatus 1 according to the invention comprises in the present case a sleeve-like housing 3, one end 4 of which is essentially closed, and the other end 5 is open. In the figures, the apparatus 1 according to the invention is shown in its most frequent position of use, in vertical arrangement, but the operating principle of apparatus 1 provides basically for operation and use in any position and, therefore, in the description end 4 is referred to as one or first or lower end 4, and end 5 is referred to as other or second or upper end 5.

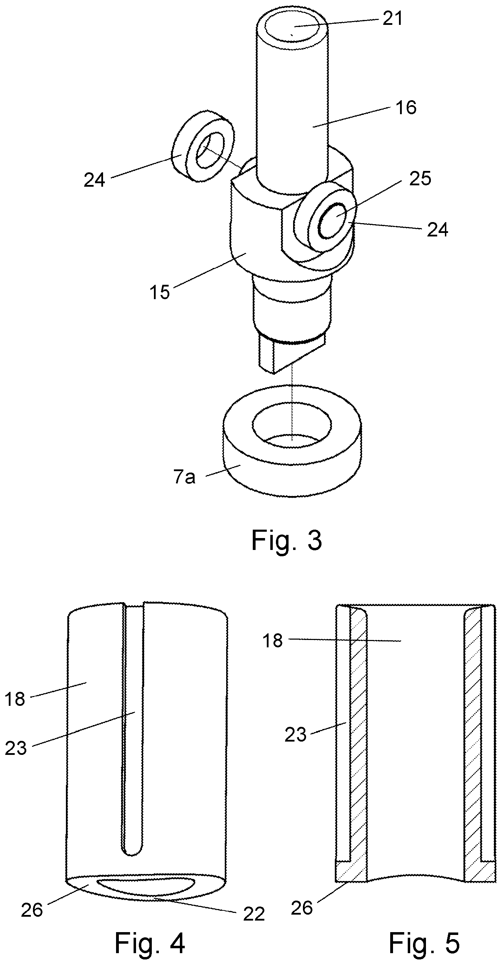

The end 4 of the housing 3 is closed by a plug 7 comprising a through opening 6. The function of a disk 8 fixed in the housing 3, connected to the side of the plug 7 towards the interior of the housing 3 is to define the position of the plug 7 on the one hand, and of a seat 12 gripping a lower part 11 of a capsule 10, arranged slideably along a longitudinal axis 9 marked by dashed line within the interior of the housing 3 in the vertical arrangement shown in the figure on the other. In the present example, seat 12 is a goblet-like cylindrical element completely filling the cross section of the housing 3, in which a centre through opening 13 is to be found, the diameter of which fits or more precisely slightly exceeds the diameter of the capsule 10 to be inserted in it, to make it possible for the capsule 10 to move along the longitudinal axis 9, and its inner chamber 14 is of such size as allows to receive and guide a stud 16 of an actuating member 15 led via a through opening 6 of the plug 7. Figure 3 shows this actuating member 15 by way of example also separately, in a perspective view. Moreover, a flexible part is arranged around the stud 16 in an interior chamber 14, consisting in the present example of a cylindrical spring 17. As can be seen, one end of the cylindrical spring 17 relies on the disk 8 and the other end is supported by the inner wall of the seat 12 sunk into the perimeter range of the chamber 14 of the seat 12, of a bigger diameter. A retainer ring 7a is also indicated, screwed in the present example into the plug 7, which provides, if necessary, for quick replacement of the actuating member 15.

Another function of the disk 8 is to ensure the moveable placement of a sleeve 18 mounted on the actuating member 15 led through the opening 6 of the plug 8. The sleeve 18 is placed on a supporting stud 19 of the actuating member 15 so that it can be pushed along it in the longitudinal direction of the actuating member 15, but non-rotatably relative to the actuating member 15. One end of the sleeve 18 relies on the actuating member 15 and its other end connects to flexible clamping means arranged in the interior of the extension 16, made up in the present example for example by a O ring 20. The diameter of the O ring 20 is also selected according to the size of the capsule 10, so as to make it possible for the lower part 11 of the capsule 10 to go through, while its end is radially positioned, if lower part 11 is already inserted, by a centring seat 21 of the actuating means 15.

The external, in the figure lower, end of the actuating member 15 isdesigned so that, if connected to a rotating structure, not shown in the drawing for the sake of simplicity but known to those skilled in the art, the rotating structure can turn the actuating member 15 around its longitudinal axis, preferably and in the present example by 90°. This plays a part in the implementation of the realisation underlying the invention, since the front surface of the end of the sleeve 18 connecting to the actuating member 15 does not extend in a plane, but in the present case it contains two wavy projections 22 designed diametrically, as can be seen in Figures 4 and 5. As can also be observed in the figures, unwanted rotation of the sleeve 18 is prevented by two diametric grooves 23 receiving inner ears of the disk 8 appropriately extending into the grooves 23.

In the present example, two bearings 24 are mounted on bolts 25 extending diametrically on the actuating member 15. The bearings 24 are arranged so that they are connected to the front surface of the sleeve 19 and thus the wavy projections22 and indentations 26 in between them designed on it. Snce the sleeve 18 can only move in the longitudinal direction of the housing 3 thanks to the disk 8, but it cannot rotate, in assembled condition, by turning the actuating member 15, the bearings 24 arranged on them move the sleeve 18 through the projections 22 and indentations 26 between them in longitudinal direction. As a result, force is exerted upon the O ring 20 located at the other end of the sleeve 18 and in contact with the end of the sleeve 18 if the sleeve 18 is moved in one direction - in the arrangement according to the figure, upwards. Snce the O ring 20 colliding with the stud 16 cannot move longitudinally in the interior of the stud 16, under the effect of force exerted on it by the sleeve 18, its diameter will change. Snce the O ring 20 diametrically completely fills the inner chamber of the stud 16, its external diameter cannot change; consequently, only its internal diameter will change, so that its interior diameter will become smaller and, if the seat 12 receives the lower part 11 of the seat 12, it will squeeze onto it in the appropriate position of the actuating member 15, thus preventing longitudinal motion of the lower part 11, i.e. its slipping out of the seat 12. This design can constitute for example the clamping means mentioned under the realisation of the invention.

Figure 2 shows the longitudinal axis of the apparatus according to Figure 1 rotated by 90°. As compared to what was shown in Figure 1, here it is possible to observe the bearings 24 mounted on the bolts 25 of the actuating member 15, and also the sleeve 18, being pressed already against the O ring 20 by the bearings24, surrounding the lower part 11 of the capsule 10 in a deformed way, squeezed onto it. In a simpler case it is also conceivable that the role of the bearings 24 is played by the bolts 25 alone but the experience is that this cheaper design may lead to the premature wear of the sleeve 18 and the bolts25.

Capsules 10, to be filled by some material preparation that is irrelevant from the point of view of the present invention, are usually manufactured so that the lower part 11 of the capsule 10 and - in case of the vertical arrangement depicted here - its upper part 27 are interconnected, but not in a way providing for complete closing, thus the upper part 27 of the capsule 10 can be removed from the lower part 11 by applying relatively small force.

In the apparatus according to the invention, thistask, i.e. the removal of the upper part 27, iscarried out by a further seat 28, an inner opening 29 of which is dimensioned so as to be suitable for receiving an upper part 27 of a capsule 10 and as the case may be part of the lower part 11 of the capsule 10. Seat 28 is designed so that it can be introduced into and removed from the open end 5 of the housing 3.

Any known and suitable structure can be used to move seat 28; in the present case, by way of example an actuator 2 is indicated that can be fitted snugly onto the upper part of the seat 28 so that it does not hinder its introduction into the housing 3. The function of the actuator 2 is, over and beyond the movement of the seat 28, to provide for the temporary holding in place of the upper part 27 of the capsule 10 placed into the opening 29 of the seat 28. In the present example, this is ensured by a vacuum unit realised in the actuator 2, not specified further, that can be realised for example by a syringe-like design of the actuator 2. The vacuum chamber 30 of the actuator 2 is connected through a duct 31 designed in the upper part of the seat 28 with the opening 29 of the seat 28 and it can hold the upper part 27 of the capsule 10 inserted into it against falling out by the depression being created. When this depression ceases, the upper part 27 can, as the case may be, slide out of the opening 29 even by itself, but the actuator 2 preferably contains also some mechanical arrangement to ensure that, e.g. a piston 32 of the syringe constituting the vacuum unit being complemented with an ejecting rod 33, which mechanically ejects the upper part 27 of the capsule 10 that may have remained there when depression ceased, i.e. during the downward return movement of the piston 32, by entering the duct 31 of the seat 28 so that the closed capsule 10 can then be delivered to desired place.

In the figure, the capsule 10 is already inserted in the apparatus 1; its lower part 11 and upper part 27 are still connected to each other and the vacuum unit of the actuator 2 is also in the default position, i.e. it does not cause depression on the upper part 27 of the capsule 10 sitting in the seat 28.

To make the lower part 11 and the upper part 27 of the capsule 10 separable, preventing the longitudinal displacement of the lower part 11 of the capsule 10 in the seat 12 must be ensured. Thiscondition is shown in Figure 2, where it is shown that the actuating member 15 rotated by 90° around its longitudinal axis relatively compresses the sleeve 19 by its bearings 24, and that exerts force on the O ring 20 which, through its deformation, gets squeezed onto the lower part 11 of the capsule 10 being hold in position. The seat 28 containing the upper part 27 can be removed from the way by the actuator 2, and the capsule 10 in the seat 12 can be filled by a material preparation by another, suitably chosen, known structure. This operation, the materials being used etc. are known to those skilled in the art, so detailing them isomitted here.

After filling the lower part 11 of the capsule 10 with some material preparation, the seat 28 is moved again by the actuator 2 to the above open end 5 of the housing 3, in a position concentric with the housing 3 and the seat 12 then it is moved in the direction of the seat 12 and sunk. Under the effect of the foregoing, the upper part 27 of the capsule 10 first gets connected with high precision and without damage to the lower part 11 of the capsule 10 - this interconnection can take place as is usual in this special field, e.g. by squeezing, snapping etc. In order not to damage the capsule 10 during this operation, in the default case, seat 12 relying on the cylindrical spring 16 can also move together with the seat 28 against the force of the cylindrical spring 16 until it collides with the disk 8. To promote the introduction of the seat 28 into the housing 3, the open end 5 of the sleeve-like housing 3 isdesigned with bevelling 34 introducing the seat 28 gripping the upper part 27 of the capsule 10, but introduction can be ensured also by e.g. an upwardly expanding conical design of the seat 28, and those skilled in the art know many solutions for that purpose.

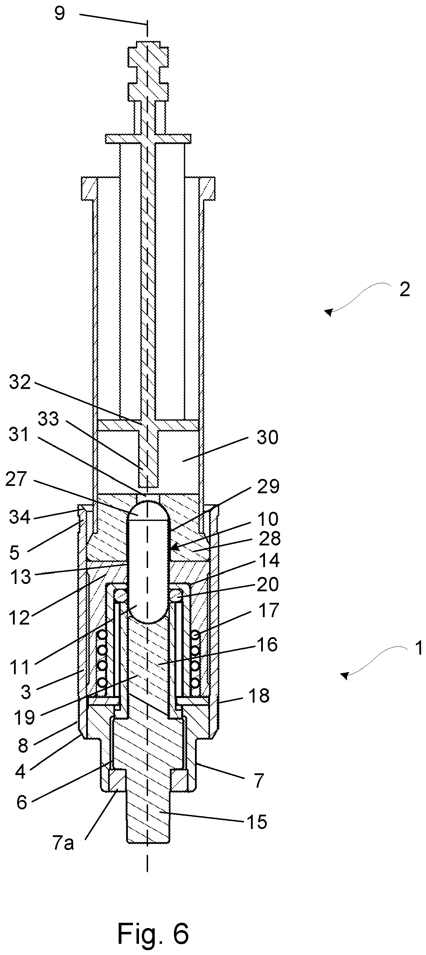

After closing the capsule 10, the actuating member 15 is turned again by 90° and, consequently, the bearings24 roll down the projections22 of the sleeve 18 into the indentations26 between the latter and, as a result, the force exerted by the sleeve 18 on the O ring 20 also ceases and the O ring 20 regains its original shape and lets the lower part 11 of the capsule 10 loose. Figure 6 shows the lower part 11 of the capsule 10, filled with material preparation already, and its upper part 27 being permanently closed: the upper part 27 has already been placed upon the lower part 11 by the actuator 2; the seat 28 containing the upper part 27 has already sunk into the open end 5 of the housing 3 so much that the upper part 27 of the capsule 10 has got fully squeezed or snapped into the lower part 11, and the seat 28 has pushed down the seat 12 against the cylindrical spring 17 so that it had sunk and collided with the disk 8.

Smilarly to Figure 5, Figure 6 shows the longitudinal section of the apparatus 1 rotated by 90°, where it is visible that the actuating member 15 rotated back to the initial position shown in Figure 1 no longer forces the sleeve 18 into its upper position, O ring 20 has already regained its original shape and no longer presses the lower part 11 of the capsule 10. While maintaining depression caused by the vacuum unit, the seat 28 and the entire capsule 10 in it can be lifted out by the actuator 2 from the inside of the housing 3 and seat 12, and it can be transported to another place according to the needs and arrangements ever in the way known to those skilled in the art, where the capsule 10 can then be removed from the seat 28 by eliminating depression and with the help of the ejecting rod 33 as the case may be.

### List of reference signs

| | | | |
|---|---|---|---|
| 1 | apparatus | 18 | sleeve |
| 2 | actuator | 19 | supporting stud |
| 3 | housing | 20 | O ring |
| 4 | end | 21 | centring seat |
| 5 | end | 22 | projection |
| 6 | opening | 23 | groove |
| 7 | plug | 24 | bearing |
| 7a | retainer ring | 25 | bolt |
| 8 | disk | 26 | indentation |
| 9 | longitudinal axis | 27 | upper part |
| 10 | capsule | 28 | seat |
| 11 | lower part | 29 | opening |
| 12 | seat | 30 | vacuum chamber |
| 13 | opening | 31 | duct |
| 14 | chamber | 32 | piston |
| 15 | actuating member | 33 | ejecting rod |
| 16 | stud | 34 | bevelling |
| 17 | cylindrical spring | | |

## Claims

1. Apparatus (1) for opening and closing two-part material containers, preferably capsules (10), comprising:
a sleeve-like housing (3) comprising a closed first end (4) and an open second end (5);
a seat (12) seizing the lower part (11) of a capsule (10) arranged slideably along a longitudinal axis (9) of the housing (3);
a seat (28) gripping the upper part (27) of the capsule (10) that can be introduced at the open end (5) of the housing (3) during the operation of the apparatus (1) and, furthermore, an actuator (2) moving the lower part (11) and the upper part (27) of the capsule (10) coaxially with the housing (3) and
along its longitudinal axis (9),
the actuator (2) being in releasable connection with the seat (28) gripping the upper part (27) of the capsule (10);
***characterized in that***
a flexible element counter-holding the seat (12) against the open end (5) of the sleeve-like housing (3) is inserted between the closed end (4) of the sleeve-like housing (3) and the seat (12) gripping the lower part (11) of the capsule (10);
the seat (12) gripping the lower part (11) of the capsule (10) comprises a centre chamber (14) open towards the closed end (4) of the sleeve-like housing (3);
at the closed end (4) of the sleeve-like housing (3) an actuating member (15) supporting the lower part (11) of the capsule (10) inserted by the actuator (2) into the capsule-seizing seat (12) gripping the lower part (11) of the capsule (10) is embedded in a rotatable manner and penetrating into the centre chamber (14) of the capsule-seizing seat (12);
flexible clamping means fixing temporarily the lower part (11) of the inserted capsule (10) is arranged in the centre chamber (14) of the seat (12) gripping the lower part (11) of the capsule (10);
a sleeve (18) is mounted unrotatably onto the rotatable actuating member (15), so that it can be pushed along the length of the actuating member (15);
the front surface of one end of the sleeve (18) isconnected to the flexible clamping means;
the front surface of the other end of the sleeve (18) comprises at least one wavy projection (22);
at least one forcing means is formed on the rotatable actuating member (15) pushing axially the sleeve (18) on a supporting stud (19) of the actuating member (15) and consequently pressing the flexible clamping means onto the lower part (11) of the capsule (10) inserted into the centre opening (13) of the seat (12) gripping the capsule (10) lower part (11) by the rotation of the actuating member (15) and the sleeve (18) relative to each other.

2. The apparatus (1) according to claim 1 ***characterized in that*** the flexible component supporting the seat (12) in the direction of the open end (5) of the sleeve-like housing (3) comprises a cylindrical spring.

3. The apparatus (1) according to claim 1 or 2 ***characterized in that*** the supporting stud (19) supporting the lower part (11) of the capsule (10) comprises a centring seat (21) radially centralising the lower part (11) of the capsule (10).

4. The apparatus (1) according to any of claims 1-3 ***characterized in that*** the flexible clamping means is an O ring (20).

5. The apparatus (1) according to any of claims 1-4 ***characterized in that*** the forcing means formed on the actuating member (15) is a bolt (25) arranged diametrically on the actuating member (15) and in connection with the front surface of the sleeve (18) comprising two diametric wavy projections (22).

6. The apparatus (1) according to any of claims 1-4 ***characterized in that*** the forcing means formed on the actuating member (15) comprises two bearings (24) in connection with the front surface of the sleeve (18) comprising two wavy projections (22) arranged diametrically on the actuating member (15).

7. The apparatus (1) according to any of claims 1-6 ***characterized in that*** the open end (5) of the sleeve-like housing (3) is formed as a bevelling (34) introducing the seat (28) gripping the upper part (27) of the capsule (10).

8. The apparatus (1) according to any of claims 1-7 ***characterized in that*** it comprises a vacuum unit connected to the seat (28) gripping the capsule (10) upper part (27) and holding the upper part (27) of the capsule (10) inserted in the seat (28).

9. The apparatus (1) according to claim 8 ***characterized in that*** the actuating member (15) comprises an ejection unit moving the upper part (27) of the capsule (10) inserted in the capsule (10) upper part (27) holding seat (28) out of the seat (28).

10. The apparatus (1) according to claim 9 ***characterized in that*** the ejection unit comprises an ejection rod (33) guided concentrically to the duct (31) providing the connection, said ejection rod (33) is connected to a plunger (32) of a syringe constituting the vacuum unit.

## Patentansprüche

1. Vorrichtung (1) zum Öffnen und Verschließen von zweiteiligen Materialbehältern, vorzugsweise Kapseln (10), umfassend:
ein hülsenartiges Gehäuse (3), das ein geschlossenes erstes Ende (4) und ein offenes zweites Ende (5) umfasst;
einen Sitz (12), der einen unteren Teil (11) einer Kapsel (10) ergreift, die verschiebbar entlang einer Längsachse (9) des Gehäuses (3) angeordnet ist;
einen, einen oberen Teil (27) der Kapsel (10) umgreifenden Sitz (28), der im Betrieb des Gerätes (1) in das offene Ende (5) des Gehäuses (3) einführbar ist,
und weiterhin einen Aktuator (2), der den Unterteil (11) und den Oberteil (27) der Kapsel (10) koaxial zum Gehäuse (3) und entlang seiner Längsachse (9) bewegt,
wobei der Aktuator (2) mit dem Sitz (28), der den oberen Teil (27) der Kapsel (10) ergreift, in lösbarer Verbindung steht;
**dadurch gekennzeichnet, dass**
ein flexibles Element, das den Sitz (12) gegen das offene Ende (5) des hülsenförmigen Gehäuses (3) drückt, zwischen dem geschlossenen Ende (4) des hülsenförmigen Gehäuses (3) und dem den unteren Teil (11) der Kapsel (10) umgreifenden Sitz (12) eingesetzt ist;
der Sitz, der den unteren Teil (11) der Kapsel (10) umgreift, eine zentrale Kammer (14) umfasst, die zum geschlossenen Ende (4) des hülsenartigen Gehäuses (3) hin offen ist;
im geschlossenen Ende (4) des hülsenartigen Gehäuses (3) ein Betätigungselement (15) drehbar und
in eine Mittelkammer (14) des die Kapsel ergreifenden Sitzes (12) hineingreifend eingebettet ist, das den unteren Teil (11) der vom Betätigungselement (2) in den die Kapsel ergreifenden Sitz (12) eingeführten Kapsel (10) stützt;
flexible Klemmmittel, die den unteren Teil (11) der eingesetzten Kapsel (10) vorübergehend fixieren,
in der Mittelkammer (14) des Sitzes (12) angeordnet sind und den unteren Teil (11) der Kapsel (10) ergreifen;
eine Hülse (18) auf dem drehbaren Betätigungselement (15) unverdrehbar montiert ist, so dass sie über die Länge des Betätigungselements (15) aufgeschoben werden kann;
die Vorderfläche eines Endes der Hülse (18) ist mit dem flexiblen Klemmmittel verbunden;
die Vorderfläche des anderen Endes der Hülse (18) mindestens einen wellenförmigen Vorsprung (22) umfasst;
am drehbaren Betätigungselement (15) ist mindestens ein Druckmittel ausgebildet, das die Hülse (18) axial auf einen Stützbolzen (19) des Betätigungselements (15) drückt und dadurch das flexible Klemmmittel durch eine Verdrehung des Betätigungselementes (15) und der Hülse (18) relativ zueinander auf den unteren Teil (11) der Kapsel (10) drückt, die in eine den unteren Teil (11) der Kapsel (10) greifende mittlere Öffnung (13) des Sitzes (12) eingesetzt ist.

2. Vorrichtung (1) nach Anspruch 1, **dadurch gekennzeichnet, dass** das den Sitz (12) in Richtung des offenen Endes (5) des hülsenförmigen Gehäuses (3) abstützende flexible Bauteil eine zylindrische Feder umfasst.

3. Vorrichtung (1) nach Anspruch 1 oder 2, **dadurch gekennzeichnet, dass** der Stützbolzen (19), der den unteren Teil (11) der Kapsel (10) trägt, einen Zentriersitz (21) aufweist, der den unteren Teil (11) der Kapsel (10) radial zentriert.

4. Vorrichtung (1) nach einem der Ansprüche 1 bis 3, **dadurch gekennzeichnet, dass** das flexible Klemmmittel ein O-Ring (20) ist.

5. Vorrichtung (1) nach einem der Ansprüche 1 bis 4, **dadurch gekennzeichnet, dass** das Druckmittel am Betätigungselement (15) als am Betätigungselement (15) diametral angeordnete zwei Bolzen (25) ausgebildet ist, die mit der einen wellenförmigen Vorsprung (22) umfassenden Vorderfläche der Hülse (18) in Verbindung stehen.

6. Vorrichtung (1) nach einem der Ansprüche 1 bis 4, **dadurch gekennzeichnet, dass** das Druckmittel am Betätigungselement (15) als am Betätigungselement (15) diametral angeordnete zwei Lager (24) ausgebildet ist, die mit der einen wellenförmigen Vorsprung (22) umfassenden Vorderfläche der Hülse (18) in Verbindung stehen.

7. Vorrichtung (1) nach einem der Ansprüche 1 bis 6, **dadurch gekennzeichnet, dass** das offene Ende (5) des hülsenartigen Gehäuses (3) als eine den, den oberen Teil (27) der Kapsel (10) greifenden Sitz (28) einleitende Abschrägung (34) ausgebildet ist.

8. Vorrichtung (1) nach einem der Ansprüche 1 bis 7, **dadurch gekennzeichnet, dass** sie eine Vakuumeinheit umfasst, die mit dem Sitz (28) verbunden ist, der den oberen Teil (27) der Kapsel (10) ergreift, und die den oberen Teil (27) der in den Sitz (28) eingesetzten Kapsel (10) hält.

9. Vorrichtung (1) nach Anspruch 8, **dadurch gekennzeichnet, dass** das Betätigungselement (15) eine Auswurfeinheit umfasst, die den oberen Teil (27) der in den oberen Teil (27) der Kapsel (10) haltenden Sitz (28) eingesetzten Kapsel (10) aus dem Sitz (28) ausbewegt.

10. Vorrichtung (1) nach Anspruch 9, **dadurch gekennzeichnet, dass** die Auswurfeinheit eine bezüglich einer die Verbindung bereitstellenden Kanal (31) konzentrisch geführte Auswurfstange (33) umfasst, wobei die Auswurfstange (33) mit einem Stößel (32) einer Spritze verbunden ist, die die Vakuumeinheit bildet.

## Revendications

1. Appareil (1) pour ouvrir et fermer des récipients de matériau en deux parties, de préférence des capsules (10), comprenant :
un boîtier (3) en forme de manchon comprenant une première extrémité fermée (4) et une deuxième extrémité ouverte (5);
un siège (12) fixant la partie inférieure (11) d'une capsule (10) disposée de manière coulissante le long d'un axe longitudinal (9) du boîtier (3) ;
un siège (28) saisissant la partie supérieure (27) de la capsule (10) qui peut être introduite à l'extrémité ouverte (5) du boîtier (3) pendant le fonctionnement de l'appareil (1)
et, en outre un actionneur (2) déplaçant la partie inférieure (11) et la partie supérieure (27) de la capsule (10) coaxialement avec le boîtier (3) et le long de son axe longitudinal (9),
l'actionneur (2) est relié de manière amovible au siège (28) qui saisit la partie supérieure (27) de la capsule (10) ;
**caractérisé en ce**
**qu'**un élément flexible maintenant le siège (12) contre l'extrémité ouverte (5) du boîtier (3) en forme de manchon est inséré entre l'extrémité fermée (4) du boîtier (3) en forme de manchon et le siège (12) enserrant la partie inférieure (11) de la capsule (10);
le siège (12) saisissant la partie inférieure (11) de la capsule (10) comprend une chambre centrale (14) ouverte vers l'extrémité fermée (4) du boîtier (3) en forme de manchon ;
à l'extrémité fermée (4) du boîtier (3) en forme de manchon, un élément d'actionnement (15) supportant la partie inférieure (11) de la capsule (10) inséré par l'actionneur (2) dans le siège (12) de saisie de la capsule saisit la partie inférieure (11) de la capsule (10) est encastré de manière rotative et
pénètre dans la chambre centrale (14) du siège de saisie de la capsule (12) ;
un moyen de serrage flexible fixant temporairement la partie inférieure (11) de la capsule insérée (10) est disposé dans la chambre centrale (14) du siège (12) et saisit la partie inférieure (11) de la capsule (10);
un manchon (18) est monté de manière non rotative sur l'élément d'actionnement rotatif (15), de sorte qu'il peut être poussé sur la longueur de l'élément d'actionnement (15);
la face avant d'une extrémité du manchon (18) est reliée aux moyens de serrage souples ;
la surface avant de l'autre extrémité du manchon (18) comprend au moins une saillie ondulée (22);
au moins un moyen de forçage est formé sur l'élément d'actionnement (15) rotatif poussant axialement le manchon (18) sur un téton de support (19) de l'élément d'actionnement (15) et pres-sant par conséquent le moyen de serrage flexible sur la partie inférieure (11) de la capsule (10) inséré dans l'ouverture centrale (13) du siège (12) en saisissant la partie inférieure (11) de la capsule (10) par la rotation de l'élément d'actionnement (15) et du manchon (18) l'un par rapport à l'autre.

2. Appareil (1) selon la revendication 1, **caractérisé en ce que** le composant flexible supportant le siège (12) dans la direction de l'extrémité ouverte (5) du logement en forme de manchon (3) comprend un ressort cylindrique.

3. Appareil (1) selon la revendication 1 ou 2, **caractérisé en ce que** le téton de support (19) supportant la partie inférieure (11) de la capsule (10) comporte un siège de centrage (21) centralisant radialement la partie inférieure (11) de la capsule (10).

4. Appareil (1) selon l'une quelconque des revendications 1 à 3, **caractérisé en ce que** le moyen de serrage flexible est un joint torique (20).

5. Appareil (1) selon l'une des revendications 1 à 4, **caractérisé en ce que** le moyen de forçage formé sur l'élément d'actionnement (15) est formé de deux boulons (25) disposés diamétralement sur l'élément d'actionnement (15) et en liaison avec la surface avant du manchon (18) comprenant des saillies ondulées diamétrales (22).

6. Appareil (1) selon l'une des revendications 1 à 4 **caractérisé en ce que** le moyen de forçage formé sur l'élément d'actionnement (15) est formé de deux roulements (24) disposés diamétralement sur l'élément d'actionnement (15) et en liaison avec la surface avant du manchon (18) comprenant des projections ondulées diamétrales (22).

7. Appareil (1) selon l'une quelconque des revendications 1 à 6, **caractérisé en ce que** l'extrémité ouverte (5) du logement en forme de manchon (3) est formée comme un biseau (34) introduisant le siège (28) saisissant la partie supérieure (27) de la capsule (10).

8. Appareil (1) selon l'une quelconque des revendications 1 à 7, **caractérisé en ce qu'**il comprend une unité à vide reliée au siège (28) saisissant la partie supérieure (27) de la capsule (10) et maintenant la partie supérieure (27) de la capsule. (10) inséré dans le siège (28).

9. Appareil (1) selon la revendication 8 **caractérisé en ce que** l'élément d'actionnement (15) comprend une unité d'éjection déplaçant la partie supérieure (27) de la capsule (10) insérée dans le siège (28) maintenant la partie supérieure (27) de la capsule (10) hors du siège (28).

10. Appareil (1) selon la revendication 9, **caractérisé en ce que** l'unité d'éjection comprend une tige d'éjection (33) guidée concentriquement au conduit (31) assurant le raccordement, ladite tige d'éjection (33) est reliée à un poussoir (32) de une seringue constituant l'unité de vide.
